# EUROPEAN PATENT APPLICATION

(11) **EP 1 900 360 A1**
(43) Date of publication of application: **19.03.2008**
(21) Application number: 07011881.5
(22) Date of filing: 16.12.1999
(51) Int. Cl.: A61K 31/00, A61K 31/4465, C12N 15/63, C07K 14/47, G01N 33/53, A61P 25/18, A61P 35/00

(54) **Homer a new target of treating psychiatric disorders**

(30) Priority: 16.12.1998 EP 98123943
(62) Divisional of application: 99965475.9
(71) Applicant: Abbott GmbH & Co. KG, 65205 Wiesbaden (DE)
(72) Inventor: Garcia-Ladona, Francisco Javier, 76870, Kandel (DE); Lang, Sandra, 67063, Ludwigshafen (DE)
(74) Representative: Grünecker, Kinkeldey, Stockmair & Schwanhäusser Anwaltssozietät

(57) **Abstract**

A method for treatment of psychosis, schizophrenia, oncological disorders, tumors and/or CNS disorders in a human being comprising administering to said human being a composition comprising an effective amount of a compound which interacts with homer or metabotropic receptors are disclosed.

## Description

Schizophrenia is a chronic psychiatric disorder affecting approximately 1% of the adult population. The economic and social cost of schizophrenia and other psychotic disorders are considerable due to the large index of hospitalization. The real causes of schizophrenia remains still unknown. The symptoms, classified as positive (hallucinations) and negative (social withdrawal, paranoia) may be observed in some cases as early as in adolescence. Schizophrenic patients suffer a progressive degradation of mood, though and cognition processes (Wright I. and Woodruff P., 1995). Compounds with a beneficial effect on the treatment of schizophrenia or psychosis have been so-called neuroleptics. Early studies suggested that an alteration of the brain dopaminergic system may be related to schizophrenic and psychotic symptoms. Although the alteration of dopaminergic function in the brain of schizophrenics is evident, whether the onset of the disease is due to this alteration or it is only a delayed consequence of the disorder remains unknown. An intense research has been developed regarding the brain dopaminergic system and the pharmacology of dopamine receptors. Typical antipsychotics, such haloperidol, with a strong therapeutic effect on the treatment of psychosis have high affinity to D2 dopamine receptors (Seeman 1987). However, this property is associated to a high incidence of extrapyramidal side effects in most of the cases in a irreversible form (Gratz S.S. and Simpson G.M. 1994; Ebadi M. and Srinivasan S.K. 1995). In addition, haloperidol have also high affinity for sigma receptors supporting them as a therapeutic target for the treatment of psychosis (Reynolds G.P. and Czudek C. 1995). Drugs specific for other brain receptors have been also proposed as antipsychotics (Fatemi H. et al., 1996). Atypical antipsychotics with mixed pharmacological profile, like clozapine, has been very useful for an effective and safer treatment of psychosis. However, to date, no fully efficient treatment have been found for the treatment of neither psychosis nor neuroleptic malignant syndrome.

It has been demonstrated that dopamine receptor blockade after acute treatment with neuroleptics induces genomic responses in brain (Deutch A.Y. 1996). In particular, transcription factors c-fos and c-jun are rapidly overexpressed and translocated to the cell nuclei leading to further genomic regulation processes. The effect seems to be related to antagonism at dopamine D1- and D2-receptors. The genomic response induced by neuroleptics may be involved not only to their beneficial effects of antipsychotics but also to their undesirable side effects.

The knowledge of gene expression changes induced by neuroleptics may help to understand both the beneficial and side effects of antipsychotic drugs and therefore also to define new and more effective targets for the treatment of schizophrenia and psychosis. The aim of the invention was to disclose genomic effects induced by neurolpetics and subsequently to identify new targets for the treatment of psychotic and neurodegenerative disorders.

Recently it has been shown that gene expression of a synaptic protein (homer) can be up-regulated by different stimulus such treatment with the neurostimulant cocaine, seizures or brain synaptic activity particularly during the development (Brakeman P.R. et al., 1997). Homer protein is new 28 kd synaptic protein which coding gene has been sequenced (Ozawa K.A. et al., 1997; Brakeman P.R. et al., 1997). The amino acid sequence contains a PDZ-domain. Homer protein shares only a 10% homology with other members of the PDZ-family thus establishing a putative new group. Homer protein is able to interact with the intracytoplasmic part of metabotropic glutamate receptor proteins mGluR1A and mGluR5 (Brakeman P.R. et al., 1997). These excitatory aminoacid receptors are coupled to excitotoxic mechanisms in brain (Knöpfel T. and Gasparini F. 1996). The precise role of homer in the central nervous system is not yet elucidated. However, the fact that homer protein contains a PDZ domain strongly suggests, as for other proteins containing such domain, the possibility of interaction with other cellular proteins involved in cell signaling systems (Pointing C.P. and Phillips C., 1995) and not only to metabotropic receptors.

The present invention provides the identification of cell systems useful for the study of homer function and as tools for the discovery of new therapeutic compounds related to this protein. The present invention provides human homer gene sequences.

The present invention provides the evidence that homer gene expression can be up regulated in vivo by the treatment with haloperidol.

The present invention also provides the partial sequence of rat and chinese hamster homer gene and the evidence that homer protein is also expressed by astrocytes.

The present invention regards the effects of antipsychotic treatment on homer gene expression and the identification of intervention targets for the treatment of schizophrenia, Tourette's syndrome, obsesive compulsive disorders, and other psychotic disorders in general. The present invention also concerns the identification of homer protein, metabotropic and sigma receptors as targets for the identification and preparation of medicinal compounds useful for the treatment of neurodegenerative processes such as senile dementia of Alzheimer's type, argirophilic grain disease and other senile dementias in general, Parkinson's disease and atypical forms of Parkinson's disease, Huntington's disease, demyelinating diseases such multiple sclerosis, progressive multifocal leukoencephalopaty, infection-induced demyelinization, and demyelinization disorders of genetic origin, amyotrophic lateral sclerosis and HIV induced dementia. The present invention also provides a new target for the treatment of CNS diseases with a evident glial cell reaction. The present invention provides new therapeutic targets for the treatment of leukemia and brain tumors.

According to a first aspect it is provided a method for treatment of neuroleptic syndrome or psychosis in a human being comprising administering to said human being a composition comprising an effective amount of a homer expression modifying compound.

A preferred embodiment relates to a method, whereby the psychosis is schizophrenia.

According to a second aspect it is provided a method for treatment of oncological disorders in a human being comprising administering to said human being a composition comprising an effective amount of a homer expression modifying compound.

According to a third aspect it is provided a method for treatment of neuroleptic induced disorders or psychosis in a human being comprising administering to said human being a composition comprising an effective amount of a compound which interacts with metabotropic receptors.

A preferred embodiment relates to a method, whereby the interaction effects an inhibition of the metabotropic receptor.

A preferred embodiment relates to a method, whereby the compound is 2-methyl-6- (2-phenylethenyl) pyridine or 2-methyl-6- (phenylethynyl) pyridine hydrochloride.

According to a fourth aspect it is provided a method for treatment of neuroleptic maligne syndrome in a human being comprising administering to said human being a composition comprising an effective amount of a compound which interacts with metabotropic receptors and/or homer.

According to a fifth aspect it is provided an isolated nucleic acid as disclosed in appendix I to 4.

According to a sixth aspect it is provided a method of screening of new compounds which modify homer expression using, Hel cells, A-172 cells, U97 cells or glial cells as described in example 3-9.

According to a seventh aspect it is provided a method of screening of new compounds modifying homer and metabotrope receptors or homer and other cell signalling proteins using glial cells, or Hel cells, or A-172 cells or U97 cells as described in examples 13,14 and 24 to 26.

According to an eighth aspect it is provided a method of treatment of CNS disorders in a human being via glial cells comprising administering to said human being a composition comprising an effective amount of a compound, which is able to act on glial cells and which is able to modulate the expression of homer.

According to a ninth aspect it is provided a method for the treatment of a disease in a human being comprising administering to the said human being a composition comprising an effective amount of a compound inducing homer protein expression or a composition comprising an effective amount of a homer peptide interacting with the homer interaction motif located in the disease-associated target.

A preferred embodiment relates to a method where the disease is degenerative disease involving cell degeneration or cell death or apoptosis and the disease-associated-target is human homologue of AFG2 protein.

A preferred embodiment relates to a method where the disease is neurodegenerative disease including ischemia and stroke and the disease-associated-target is insulin like growth factor binding protein.

A preferred embodiment relates to a method where the disease is hepatic degenerative processes and the disease-associated-target is interleukin 6 binding protein.

A preferred embodiment relates to a method where the disease is tissue degenerative processes involving cell death or apoptosis including neurodegenerative disease and ischemia-induced degeneration and the disease-associated-target is cytochrome oxidase or cytochrome P450 XIA1 or topoisomerase I.

A preferred embodiment relates to a method where the disease is human diseases including brain diseases and tumour progression and the disease-associated-target is GPI-linked NAD-arginine ADP-ribosyltransferase.

A preferred embodiment relates to a method where the disease is metabolic disorder including obesity and the disease-associated-target is pyruvate carboxylase.

A preferred embodiment relates to a method where the disease is associated to cholesterol production including senile disorders and the disease-associated-target is low density lipoprotein receptor related protein.

A preferred embodiment relates to a method where the disease is a human neurodegenerative disease and the disease-associated-target is human F-spondin.

A preferred embodiment relates to a method where the disease is herpes simplex infection and propagation and the disease-associated target is DNA helicase/primase complex associated protein.

A preferred embodiment relates to a method where the disease is herpes simplex virus infection and propagation and the disease associated-target is UL56 protein.

A preferred embodiment relates to a method where the disease is varicela zoster virus infection and propagation and the disease associated-target is serin/threonine-protein kinase.

A preferred embodiment relates to a method where the disease is sarcoma virus infection and propagation and the disease-associated target is sarcoma virus receptor.

A preferred embodiment relates to a method where the disease is Japanese encephalitis virus infection and propagation and the disease associated-target is NS proteins.

A preferred embodiment relates to a method where the disease is bovine immunodeficiency virus infection and propagation and the disease-associated-target is virion infectivity factor (factor Q).

A preferred embodiment relates to a method where the disease is pox virus infection and propagation and the disease-associated target is protein A11.

A preferred embodiment relates to a method where the disease is trypanosomiasis and the disease-associated-target is retrotransposable element slacs 45 kd protein.

A preferred embodiment relates to a method where the disease is propagation and infection of candida albicans and the disease-associated-target is topoisomerase 1.

### Regulation of homer gene expression by haloperidol

### Methods

### Animals

Adult Sprague-Dawley rats (250 g) were maintained in normal environmental conditions with free access to food and water ab libitum.

### Treatment

Animals were treated with haloperidol (0.5 and 5 mg/kg) or saline. Naive (non-treated) animals were used as additional controls. Animals were also treated with amphetamine or with amphetamine and MPEP or SIB-1893 as described in example 18b.

### Example 1. Tissue preparation.

The animals were sacrificed by decapitation 90 minutes after treatment. Whole brain was rapidly removed from the skull, frozen in dry ice and stored at -30°C. Rat brain sections (15 µm) were obtained at -20°C in a cryostat, mounted in gelatine-coated slides and stored at -30°C until used.

### Example 2. Synthesis and labelling of oligonucleotides

Olygonucleotide sequences 40 base length were selected from the homer rat gene sequence published in the GENEBANK (accession number: U92079). Antisense oligonucleotide homerAT (5'-CTCGAGTGCTGAAGATAGGTTGTTCCCCCATTTTG-CCCA-3') was complementary to bases 559 to 599. Antisense oligonucleotide homerBT (5' -GTTCCATCTTCTCCT-GCGACTTCTCCTTTGCCAGCCGAGC-3') was complementary to bases 894-934. The corresponding sense oligonucleotides (homerAS and homerBS) were used as control probes. Synthesis was performed in a 395 Applied Biosystems DNA Synthesizer. Purified oligonucleotides were dissolved in DEPC-treated water and stored at -30°C until use. Labelling of synthetic oligoprobes was performed using a deoxynucleotidyl-transferase (TdT) labelling kit (NEP-100, NEN, Bad-Homburg, Germany). Briefly, 5 pmol of probe were incubated (2 hr) with TdT (36 units) in presence of 50 pmol of (³⁵S] dATP (NEN) and Cl₂Co. The reaction was stopped and the labelled oligonucleotides purified by column chromatography. Labelling efficacy was checked by paper chromatography in phosphate buffer system.

### Example 3. In situ hybridization histochemistry

Homer gene expression in rat brain was studied using in situ hybridization techniques well known in the art. In situ hybridization was performed as previously described (Garcia-Ladona et al., 1994). Briefly, Tissue sections were fixed with paraformaldehyde in PBS and treated (min) with pronase (0.25 mg/l), rapidly washed, dehydrated by consecutive incubation with 60%, 80%, 90% and 100% ethanol, rapidly dried and used for hybridization. Brain sections were incubated with 100 µl of 10 mM Tris-HCl hybridization buffer (pH: 7.5) containing 50% formamide, 0.6 M NaCl, lx Denhardt's solution, 1 mM EDTA, 0.58 mg/ml yeast t-RNA, 10% dextran sulphate, 10 mM DTT and [³⁵S]-labelled oligoprobe (13500 c.p.m./µl). A nescofilmR strip was deposed over hybridization mixture to avoid evaporation and the slides incubated overnight at 42°C in a humid chamber. Afterwards, hybridization solution was washed out from the slides and non specific hybridization was eliminated by incubation (4x 1 hr) with 10 mM Tris-HCl ph:7.5 containing 0.6 M NaCl and 1 mM EDTA at 60°C. Nucleic acids in the sections were precipitated by two consecutive washes with 70% and 95% ethanol containing 0.3 M ammonium acetate. The slides were dried and expossed with a X-0-mat X-ray film (Kodak). The films were developed after 48 hr. Measurements of autoradiographic images were performed with an image analysis system equipped with SigmaScanpro software (Jandel Scientific).

### Example 4. Cell culture methods

The culture of HEL cells was performed using conditions commonly used by the art. Cells were grown in RPMI media containing serum (10%), penicilin (90 unit/ml) and streptomycin (90 mg/ml).

Culture conditions were 95% humidity and 5% CO₂. Cell population was splited 1/3 every 3 days.

The culture of glioma A-172 cells (ATCC) was performed using conditions commonly used by the art. Cells were grown in medium containing special supplement (DMEM NUT F-12) containing serum (10%), penicilin and streptomycin. Culture conditions were 37°C, 95% humidity and 5% CO₂. Cells were grown till confluence and then splited.

The culture of glioma U87 cells (ATCC) was performed using conditions commonly used by the art. Cells were grown in medium containing special supplement (DEMEM NUT F-12) containing serum (10%), penicilin and streptomycin. Culture conditions were 37°C, 95% humidity and 5% CO₂. Cells were grown till confluence and then splited.

### Example 6. Culture of glial cells

The culture of glial cells was performed by methods clearly described in the art. Whole brains from new born rats were dissected out of the skull in aseptic conditions. Brain areas (striatum, cortex and hippocampus) were dissected and immersed in culture medium without serum. Small pieces of tissue were obtained by scretching with micro forceps. Tissue was homogenated by 10 passages through a 1.2 mm gauge. Cell suspensions were centrifugated and the pellet resuspended in culture medium and plated in pedri dishes. Cell were incubated for 2-3 hr at 37°C, 95% humidity, 5% CO₂. Non-attached cells were aspirated and centrifugated. Pelleted cells were resuspended in medium containing 10% serum and antibiotics and then plated in petri dishes for 10-20 days. Medium was renewed every 3-4 days.

### Example 7. RNA isolation

The isolation of RNA from different cell and tissue sources was performed using a single-solution extraction method commercially available (Trizol^{®}, Gibco Life Sciences). The cells were washed with RNAse free PBS and the homogenized with Trizol^{®}. The total RNA present in each sample was determined by measuring light abosrbance and extrapolating to a calibration curve.

### Example 8. RT-PCR methods

Homer gene expression in human glioma cells (A-172, U87), in brain areas, in glial cell cultures, and in HEL cells was studied using reverse transcriptase and polymerase chain reaction methods of common use in the art. A commercially available RT-PCR kit was used (Ready to go, Amersham/Pharmacia Biotec). Protocols were adjusted to the supplier recommendations. RT-PCR was performed using 1 mg total RNA. Primers were selected by analysing the homer gene sequence (accession number: U92079) logged in the GENEBANK. Thermocyler was programmed as follows (RT 30 min at 42°C, PCR 23 cycles of 1 min 95°C, 1 min 60°C, 2 min 72°C). At the end of PCR cycles, samples of PCR mixture were electrophoresed in agarose gels as described by commonly used protocols.

### Example 9. Analysis of nucleic acids by gel electrophoresis

Gels for the analysis of RT-PCR products were prepared by melting agorose (1%) in electrophoresis buffer (Current Protocols in Molecular Biology, John Wiley & Sons, 1995) at 60°C. PCR samples were mixed with sample buffer containing and loaded (1µg/lane). Electrophoresis was run 60 min. and separation of fragments was checked by u.v illumination.

### Example 10. Analysis of gene sequences

The analysis of gene sequences obtained by RT-PCR was performed by using software commercially available or in the public domain. The sequence identification was performed by homology search using DNASIS software (HITACHI) and software available in the public GENEBANK.

### Results

Homer gene expression is up regulated after antipsychotic treatment.

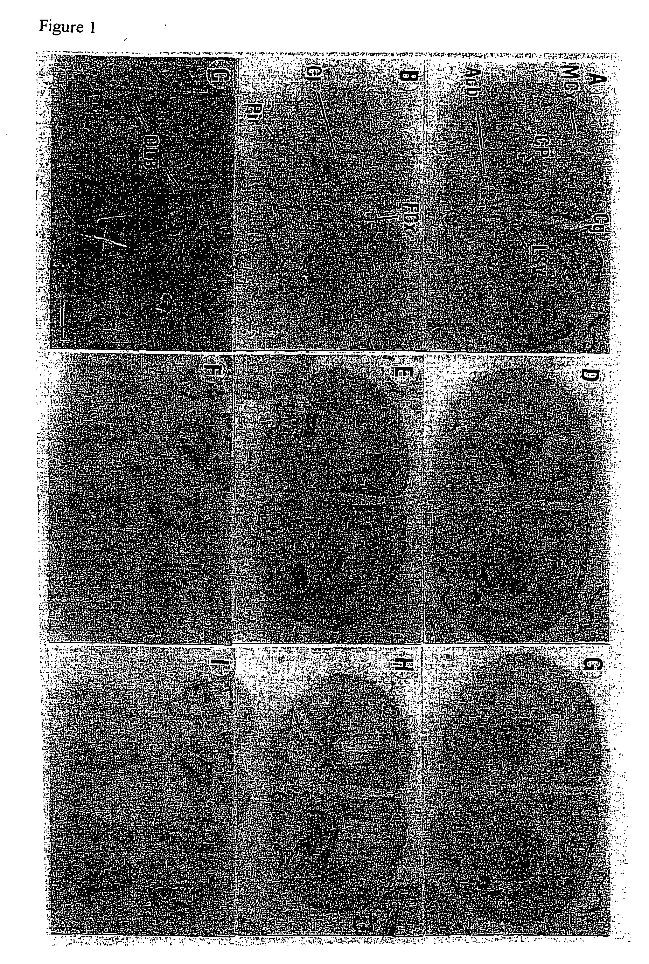

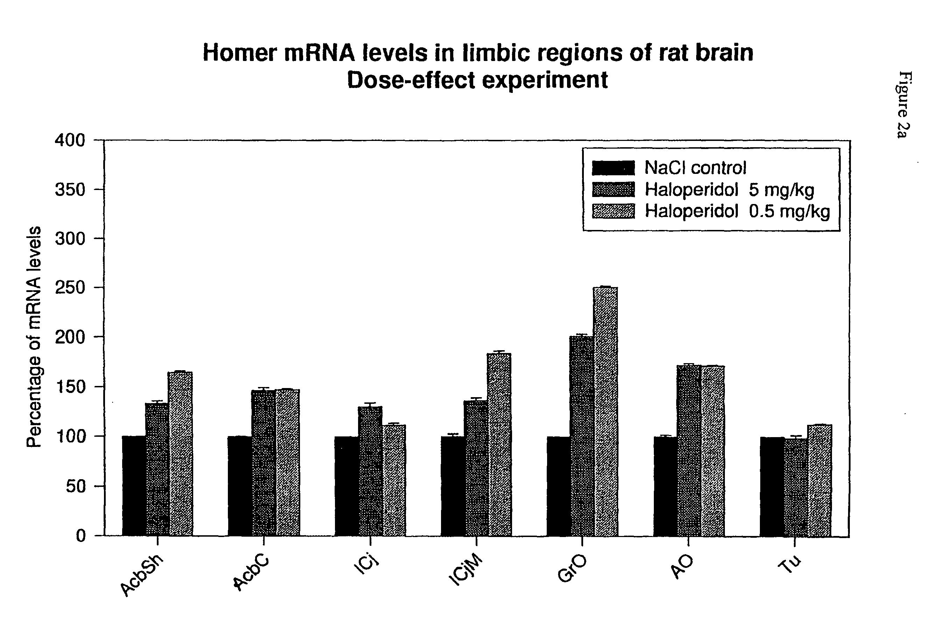

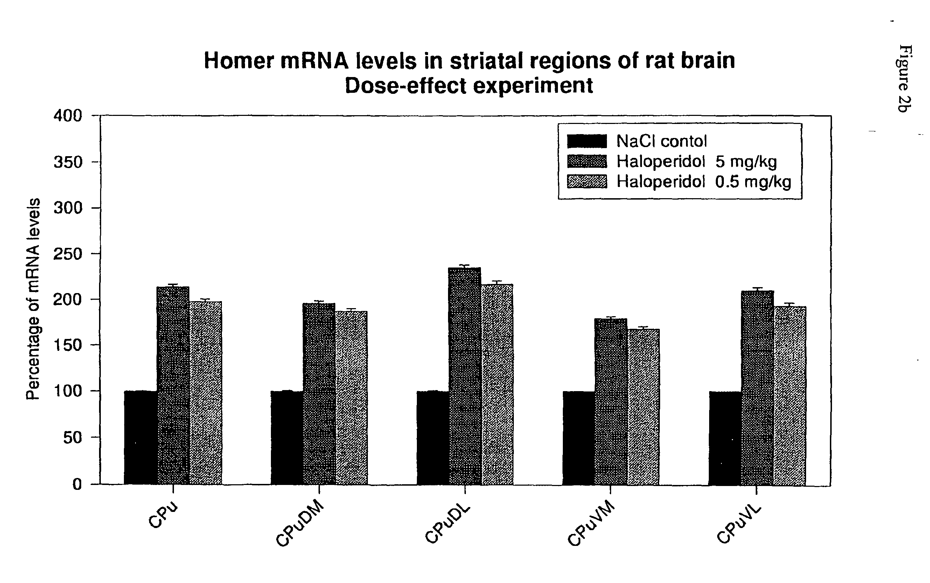

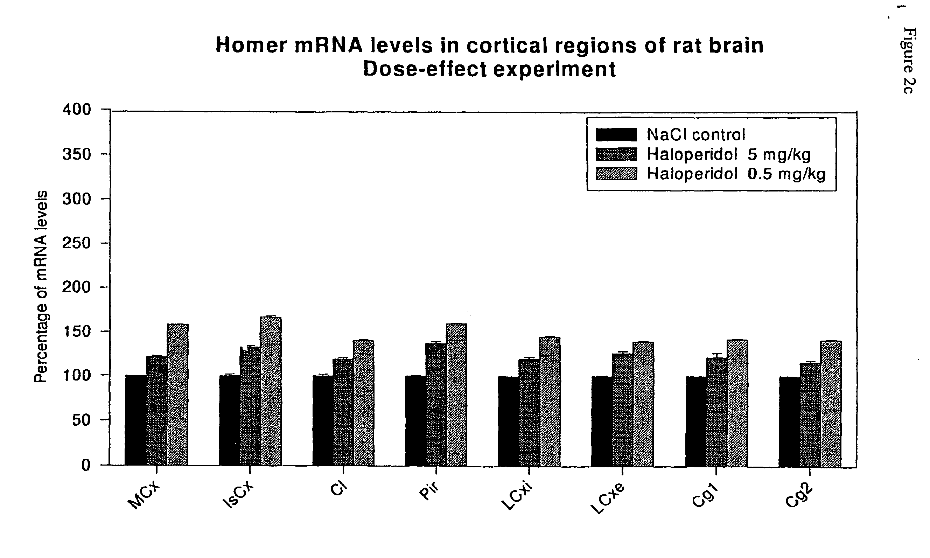

The in situ hybridization images showed that homer mRNA transcritps were present in higher levels in haloperidol treated animals than in controls (Fig. 1) The differences, on optical density measured in autoradiographic films, between control and treated animals are shown in figure 2. Homer gene expression induced by treatment with amphetamine is reduced in brain frontal cortex by administering compounds MPEP and SIB-1893.

### Homer gene is expressed by neurotumoral cells

The fragments of DNA obtained after RT-PCR using RNA from A-172 and U87 cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see apendix 1).

Homer gene is expressed by Chinese Hamster Ovary Cells.

The fragments of DNA obtained after RT-PCR using RNA from CHO cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see appendix 1).

Homer gene is expressed by human eritroleukemic (HEL) cells.

The fragments of DNA obtained after RT-PCR using RNA from HEL cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see appendix 1)

### Example 11 provides a method to detect the efficacy of antisense oligonucleotides in cultured cells.

Cells were cultured as described in example 4. Cells were treated for different times with different concentrations of antisense, sense and missense oligonucleotides complementary to human homer gene sequences. The presence of homer protein were determined by western blots (example 13) and immunocytochemistry (example 12) using specific antibodies directed against human homer polypeptide sequences. The effects of antisense oligonucleotides complementary to human homer gene sequences were determined by using different methods of to determine second messenger signal pathways activation (see examples 14, 15 and 16)

### Example 12 provides a method to detect homer protein by immunocytochemistry. The method was similar to that previously described (Garcia-Ladona 1997)

### Example 13 provides a method to detect homer protein by using western blots. The method was similar to that described previously (Garcia-Ladona 1997)

### Example 14 provides a method to determine the agonist-induced phospholipase C activity.

The method was basically described previously (Garcia-Ladona et al., 1993). Cells were incubated for 24 hr with 0.125 µM [³H]myoinositol. Non incorporated [³H]myo-inositol was eliminated from the medium and replaced with Krebs-Henseleit buffer containing 10 mM LiCl. After 10 min incubation, different agonist were added for 45 min. The reaction was stopped by replacing the stimulation medium with distilled water. In the case of tissue samples, the procedure is very similar (Garcia-Ladona et al., 1993) Cells were frozen and stored at -80°C. Production of [³H]myo-inositol monophosphate was determined in cell samples by known methods of chromatographic purification (in Methods in Neutrotransmission receptor analysis. Eds H.I. Yamamura, S.J. Enna M.J. Kuhar, Raven Press, 1990). A similar method to determine agonist-mediated phospholipase C stimulation was used by preparing membrane fractions and incubating with [³²P]PIP₂ and agonist or antagonists. In this case the production of IP3 was determined. Methods known of the art have been also optimized for using microtiterplates based systems. Commercially available materials allows to perform high throughput and secondary screening (Amersham pharmacia biotech and NEN).

Example 15 provides a method to determine agonist-induced elevation of intracellular Ca⁺⁺ levels.

The method used was similar to known methods described in the literature (Nuccitelli R, 1994). Briefly cells were grown in culture bottles as indicated (example 4). Cells were softly scraped before reaching confluence. Cell were labelled with Fura-2 by incubating (30 min) with Fura-2-acetyl-methylester at room temperature. Cells were centrifuged at 180 x g for 10 min and resuspended in DMEM-F 12 medium without serum and incubated at 37°C, 5% CO₂ and 95% humidity for 45 min. Intracellular calcium levels were determined in a fluorescence microscope equipped with an appropriate filter exchange system ( Olympus / Hamamtsu ). Fluorescence ratio ( A340 / A380 ) was measured using Argus^{®} software. Intracellular calcium levels were monitored in single cells for a short period in the absence of drugs and then for 30 min after the addition of selected compounds.

Example 16 provides a method to measure agonist-induced cAMP production in cultured cells.

The method was similar to that used currently in the art (In Methods in Neurotransmission receptor analysis. Eds H.I. Yamamura, S.J. Enna, M.J. Kuhar, Raven Press, 1990). Briefly, cells were incubated for 10 min in culture medium in the absence of serum and antibiotics. Reaction was stopped by heating to 95°C 15 min. Cell samples were frozen and stored at -80°C. cAMP levels were determined with commercial available kits (Biotrak from Amersham) using the CAMP binding protein. Methods known of the art have been also optimized for using microtiterplates based systems. Commercially available materials allows to perform high throuphput and secondary screening (Amersham and NEN).

Example 17 provides a method to determine the effects of antisense oligonucleotides in vivo.

Antisense nucleotides were dissolved in physiological saline and injected intravenously and intracerebroventricularly in animals. Different periods of treatment were established. After treatment, animals were sacrificed and different organs and body fluids used for histological analysis. Animals were used to determine the effects on the homer and other cellular proteins production by using immunohistochemical, immunocytochemical and western blot methods described in the examples 12 and 13 respectively. Animals were also used to determine the effect on cell signalling processes by methods exemplified in the examples 14 and 16. A group of animals were tested in behavioural models for antipsychotic effects (see examples).

Example 18a provides a method to determine the efficacy of a compound in an animal model for prediction of antipsychotic activity.

The method have been reported in the literature by injecting PCP in animals. Animals were treated before and after PCP with different doses of compound. Afterwards a set of animals were used in behavioural models predictive of psychotic activity to asses compound's efficacy (example 28). An additional set of animals may be used for the analysis of different receptor activity as described in example 14 and 17.

### Example 18b:

Methamphetamine antagonism was tested by recording methamphetamine-induced hyperactivity (measurement of locomotor activity).

Mice (NMRI, 21-26 g; female) received drug or vehicle, intraperitoneally, 30 min prior to methamphetamine (MET, 1 mg/kg po). Locomotor activity was recorded in cages equipped with light beams (2 mice/cage/dose) for 1 h, starting 30 min after MET. For calculation of drug effects the counts recorded during the time period of 15 to 60 min after start of the measurement were selected. The control value was calculated as the difference between the counts recorded for the methamphetamine group and the vehicle-treated group during the same time period.

### Cataleptogenic effects

The cataleptic syndrome was tested according to the method described by Wirth et al. (Arch. Int. Pharmacodyn. Ther. 115, 1-31, 1958). The animals (male rats, Sprague-Dawley bodyweight 210-225 g; n/dose=4) were regarded as cataleptic if they remained in an abnormal posture for more than 15 sec, i.e. one foreleg an a 9-cm-high piece of cork. The animals were tested 30m 60, 120, 180 and 300 min after intraperitoneal administration of the test compound.

### Results

### Table

| Compound | Methamphetamine antagonism ED50 [mg/kg ip] | Cataleptogenic effect [x/n] at dose [mg/kg ip] |
|---|---|---|
| BSF 470213 | 53.4 | 0/4 at dose 100 |
| BSF 470214 | 51.2 | 0/4 at dose 100 |

The test compounds showed a dose-dependent antagonism of methamphetamine-induced hyperactivity. No induction of catalepsy was found.

The compounds used are SIB 1893 (2-methyl-6-(2-phenylethenyl)-pyridine = BSF 470213 and MPEP (2-methyl-8-(phenylethynyl)-pyridine hydrochloride = BSF 470214.

### Example 19 provides a method to determine the efficacy of compounds on preventing neuroleptic induced malignant syndrome.

Different animals models are may be used including haloperidol induced catalepsy and chronic treatment with haloperidol. Animals may be subsequently used to determine behavioural deficiencies (example 28, anatomical neurodegeneration and changes in gene expression (as exemplified in examples 1-3, 12, 13).

Example 20 provides a method to determine the efficacy of a compound in the treatment of demyelinating diseases. Different animals models of demyelinization are known of the art. Demyelinization was induced by injecting antibodies. Animals were treated with the compounds after the induction of myelin loss. Animal brains were used to determine the levels and integrity of myelin.

Example 21 provides a method to determine the efficacy of a compound in the treatment of demyelinizating diseases.

The method consist in the use of oligodendrocytes-enriched cell cultures from normal and demyelinizated animals (jimpy mutation) as described (Garcia-Ladona et al., 1997). Cells were treated with different doses of the compound and the integrity of myelin sheets and the levels of myelin markers were determined.

Example 22 provides a method to predict efficacy of a compound in Parkinson's disease. Different models were used, MTP induced Parkinsonism in mice, 6-OHDA induced degeneration in substantia nigra (Drug Discovery and Evaluation, Eds. H.G. Vogel and W.H. Vogel 1997).

Example 23 provides a method to determine the beneficial effects of a compound in senile dememntia of Alzheimer type.

The method is known of the art and consists on the use of transgenic animals overproducing b-amyloid protein. Animals can be treated with compounds and analysed for memory deficits and other behavioural parameters.

Examples 24 provides a method to identify compounds with high affinity to metabotropic receptors. Similar methods have been extensively described in the literature (Drug Discovery and Evaluation, Eds. H.G. Vogel and W.H. Vogel 1997).

Binding saturation kinetics of a radioligand. Membranes (200 µl) were incubated (600 µl total volume) in 100 mM Tris-HCl (pH: 7.7) containing 1 mM EDTA (buffer B) with increasing concentrations of radioligand in the presence (non specific binding) or in the absence (total binding) of an antagonist at high concentration. Incubation was prolonged for 90 min at 30°C; afterwards, samples were filtered, using a Skatron filtration system, through GF/B filters embedded in 0.3% poly-ethylenimide. Filters were washed with 9 ml of butter B at 4°C. Radioactivity retained in the filters was measured by liquid scintillation counting using 5 ml Ultima-Gold.

Displacement of radioligand binding: Binding displacement experiments were performed basically as reported in other studies. Membranes (200 µl) were incubated in buffer B (600 µl total volume) with increasing concentrations of the selected compounds in the presence of a selected concentration of radioligand. After a 87 min incubation period at 30°C, samples were filtered with buffer B at 4°C through GF/B filters. Filters were washed with 9 ml buffer B. Radioactivity retained in the filters was determined as above. Total binding was defined as radioligand binding observed in the absence of other compounds. Non specific binding was defined as radioligand binding levels observed in the presence of antagonist in high concentration.

Analysis of radioligand binding data Saturation parameters radioligand were estimated both by no-linear regression analysis and from linear plots by using SigmaPLot software (Jandel Scientifivc Germany). Displacement curves were build from radioactive binding levels expressed as percentage of total binding. IC₅₀ and Hill coefficients (n_{H}) were estimated by non linear regression analysis.

Example 25 provides a method to identify compounds with agonist activity at different receptors by measuring agonist stimulated [³⁵S]GTPγS binding.

The methods are very well known in the art (Hilf and Jakobs 1992). Briefly, agonist activity was determined by measuring drug-induced changes of [³⁵S]GTPγS binding in membranes from cells. Cell membranes were obtained as indicated above. [³⁵S]GTPγS binding assay was performed using a previously described method with minor modifications. Cell membranes (12 µg) were incubated with 50 mM trietanolamine-HCl buffer (pH: 7.5) containing 6.75 mM MgCl₂, 150 mM NaCl, 1 mM DTT, 1 mM EDTA, 10 µM GDP and [³⁵S]GTPγS (nM). Following 60 min incubation, at 30°C, in the absence or in the presence of different drug concentrations, the assay mixture (100 µl) was rapidly filtrated through GF/B filters using a Skatron^{®} filtration device. Filters were quickly washed with 9 ml of 50 mM Tris-HCl buffer (4°C) containing 100 mM NaCl, 5 mM MgCl₂ at pH: 7.5. Radioactivity retained in the filters was determined by scintillation spectrometry using Ultima-Gold scintillation liquid. Drug activities were expressed as % of basal binding levels measured in the absence of the compound. Curves were fitted using a non-linear regression analysis software (Sigma Plot, Jandel Scientific, GErmany) to the general equation E = (L*Eₘₐₓ)/(L+EC₅₀) where E is the effect, L is ligand concentration, Eₘₐₓ is the maximal effect and EC₅₀ is the concentration inducing 50% of the maximal effect.

### Example 26 provides a method to prepare cell membranes.

Different methods have been described in the art (Biologiacal Membranes, Eds). Membranes were prepared form cell cultures. Cells were softly scraped from bottle surface and centrifuged 10 min at 180 x g. Cell pellets were resuspended in 5 mM Tris HCl buffer (pH: 7.6), containing 5 mM EDTA, 5 mM EGTA, 0.1 mM PMSF and 3 mM benzamidine (buffer A) and incubated for 15 min at 4°C. Cell suspension was homogenized (6 x 3s) in an Ultraturrax (15000 r.p.m.) and centrifuged for 1 min at 1000 x g and 4°C. Nuclear pellet was resuspended in buffer A, homogenized and centrifuged as above. Supernatants of both steps were collected and centrifuged for 20 min at 40000 x g at 4°C; pellet was resuspended in buffer A and homogenized (1 x 15s). Membrane suspension was centrifuged for 20 min at 40000 x g at 4°C. The resulting pellet was resuspended in buffer A containing 10% glycerol and 1% bovine serum albumin. Aliquots were frozen and stored at -80°C until use.

### Example 28 provides a method to study psychosis by using animal models.

The method has been described in the literature (Swerdlow N.R. et al., 1996). Animals may be treated by different routes with compounds and checked for behavioural parameters to determine efficacy as antipsychotic agents (Drug Discovery and Evaluation, Eds H.G. Vogel and W.H. Vogel, 1997).

Example 29 provides a method to study psychosis by using an animal model regarding neonatal lesion.

The method is very weel known of the art (Lipska et al., 1993). Animals are lesioned with a excitotoxin in the central hippocampal area in the neonatal period and are used in the adulthood. Animals may be treated with compounds and checked for different behavioural parameters including prepulse inhibition paradigm (example 28).

The term human homer gene refers to polynucleotide sequences with homology to the so-called rat homer gene. The term human homer protein refers to a peptide resulting of the translation of human homer gene sequences using the natural code.

The present invention provides four partial nucleotide sequences of human homer gene.

The present invention also provides partial nucleotide sequences of homer gene of hamster and homer protein expressed by astrocytes in culture.

Nucleic acid sequences according with the present invention can be used to design anti-sense oligonucleotides and to determine aminoacid sequences of polypeptides encoded by them.

Modified antisense oligonucleotide synthesis is well known in the art (Gene Therapy, Eds, J.T. August, 1997). Different oligonucleotide modifying groups can be used.
Modified anti sense oligonucleotides will be tested to determine time-life, bioavailability an efficacy on inhibiting the homer protein translation (examples 11, 12 and 13).

The human homer peptides of present invention can be used to raise specific antibodies. The present invention also includes the use of antibodies or antisense oligonucleotides raised against human homer protein as therapeutic compounds and as probes to detect human homer protein and gene respectively (see examples 1, 3, 12 and 13). Where probes means unlabelled or isotope or non-isotopically labelled compounds that bind to a specific target. The antibodies against the human homer sequence can be obtained using the known protein chemistry techniques.

The present invention also includes a method to disclose the role of homer protein in neurotumoral and leukemic cells for example in invasive activity, proliferation, cell survival, apoptosis, signal transduction, genomic activity toxicity, sensibility to infectious agents and biological and chemical compounds.

The present invention includes a method to study the role of homer protein on the activity of other cell signalling mechanisms by using HEL cells and A-172 and U97 human glioma cells.

The present invention includes a method to study the role of human homer protein on the activity of specific cell proteins and receptors by transfecting their genes in HEL cells, A-172 and U87 human glioma cells and CHO cells. Tranfection techniques are well known of persons skilled in the art and involve the transference into the cell of gene sequences included in a vector. Where vector means polynucleotide sequences that facilitate the insertion in a host of a given genetic information. Where vectors include plasmids and eucaryotic viruses and bacteriophages. Many vectors and expression systems are well known and documented in the art (for example pcDNA3, pCR2.1 from Invitrogene).

### Methods to detect human homer

The present invention includes a method to detect human homer protein in human brain by using antisense oligonucleotides or antibodies indicated above. Examples of such methods are reported in examples 1, 2, 3, 12 and 13).

The present invention provides a method to detect human brain (glial) tumors by using antibodies directed against human sequences of homer or using isotope- or non-isotopically-labelled oligonucleotide probes complementary to human homer sequence. Methods are exemplified in examples 1, 2, 3, 12 and 13.

The present invention includes a method to detect human glioblastome and leukemic cells in culture using antibodies directed to human homer protein or antisense nucleotides complementary to human homer protein gene sequences. Methods for such detection are reported in examples 2, 3, 4, 12 and 13.

### Neurodegenration and homer

The present invention includes a method to treat human brain degenerative processes by using compounds modifying homer gene expression. Methods to identify such compounds are exemplified in the examples.

Where degenerative processes are ischemia of vascular origin, ischemic states induced by brain or spinal cord trauma, epilepsy, psychotic disorders including schizophrenia, senile dementia including senile dementia of Alzheimer's type, demielynating diseases, HIV induced dementia and neurodegeneration involving excitatory aminoacid receptors and neurodegeneration involving reactive glial cells. Degenerative processes is a term used also as synonym of neurodegeneration and of neurodegenerative disease.

The present invention also provides a method for treating or prevent neurodegeneration by using compounds facilitating (modifiying) the interaction between homer and other components of the cell signalling pathways, genetic information or cellular proteins. Where genetic information is any DNA or RNA sequences present in the cell.

The present invention also provides a method for treating or prevent neurodegeneration by using compounds facilitating the interaction between homer and metabotropic receptors. Such compounds may be identified by coincubating membranes from cells expressing metabotropic receptors, purified human homer protein, antibodies directed to homer protein and by using a commercial methods (SPA, Amersham or flashplates) to detect binding activities.

The present invention also provides a method for treating or prevent neurological deficits observed in patients suffering of neurodegenerative diseases by using agonist/antagonist of metabotropic receptors. Such compounds may be identified using current membrane binding methods as described.

The present invention also provides a method for treating or prevent neurological deficits observed in patients suffering of schizophrenia or any other psychotic disorders by using antagonists of metabotropic receptors. Such compounds will be identified using membrane binding methods described in the examples. Suitable compounds are those used in example 18b.

The present invention also contains a method to treat and prevent neurological deficits induced after treatment with typical antipsychotics by using antagonist/agonist of metabotropic receptors. Such compounds will be identified using membrane binding methods described in the examples.

The present invention contains a method to modify the expression of homer protein by using compounds with affinity to sigma or dopaminergic receptors. The compounds will be selected by using in the examples. Their efficacy will be determined by using the methods described in examples 1, 2, 3, 6, 7, 8 and 9.

### Brain tumors and leukemias

The present invention provides a method to treat human brain (glial) tumors by using modified or unmodified antisense oligonucleotides complementary to human homer mRNA sequences or by using antibodies directed against human homer protein, or by using compounds modifying the expression of homer protein acting directly in the transcription or in the translation, protein folding, protein maturation, protein turnover processes, or by using compounds that modify the interaction between homer and any other cellular protein or peptide included those involved in signalling processes, and genetic information. Where genetic information is DNA or RNA sequences. Compounds may active by different treatments including intravenous application, orally treatment or stereotaxically injected in the brain tumor area.

The present invention provides a method to treat leukemias by using modified or unmodified antisense oligonucleotides complementary to human homer mRNA sequences or by using antibodies directed against human homer protein, or by using compounds modifying the expression of homer protein acting directly in the transcription or in the translation, protein folding, protein maturation, protein turnover processes, or by using compounds that modify the interaction between homer and any other cellular protein or peptide included those involved in signalling processes, and genetic information. Where genetic information is DNA or RNA sequences. Compounds may active by different treatments including intravenous application and oral treatment.

Another subject of the invention is a method for treatment of specific diseases by administering to a human being in need thereof a composition which comprises an effective amount of a compound which induces the homer protein expression. The so induced homer expression products interact with targets which are associated with the respective disease. Another embodiment for the interaction with the disease-associated-target is to administer polypeptides comprising a sequence form the homer expression product. These polypeptides (homer peptides) interact with the homer interaction motif of the respective disease-associated target. The diseases and the corresponding disease-associated targets are disclosed in the claims. 
Appendix 1
Sequence of homer gene amplified from HEL mRNA and its correspondign
aminoacid sequence

A CTCGAGCTCA TGTCTTCCAA ATTGACCCAA ACACAAAGAA GAACTGGGTA
CCCACCAGCA AGCATGCAGT TACTGTGTCT TATTTCTATG ACAGCACAAG AAATGTGTAT
AGGATAATCA GTTTAGATGG CTCAAAGGCA ATAATAAATA GTACCATCAC CCCAAACATG
ACATTTACTA AAACATCTCA GAAGTTTGGC CAGTGGGCTG ATAGCCGGGC AAACACCGTT
TATGGATTGG GATTCTCCTC TGAGCATCAT CTTTCGAAAT TTGCAGAAAA GTTTCAGGAA
TTTAAAGAAG CTGCTCGACT AGCAAAGGAA AAATCACAAG AGAAGATGGA ACTTACCAGT
ACACCTTCAC AGGAATCCGC AGGCGGGGAT CTTCAGTCTC CTTTAACACC GAAAGTA

STRAHVFQID PNTKKNWVPT SKHAVTVSYF YDSTRNVYRI ISLDGSKAII NSTITPNMTF
TKTSQKFGQW ADSRANTVYG LGFSSEHHLS KFAEKFQEFK EAARLAKEKS QEKMELTSTP
SQESAGGDLQ SPLTPKVXG

Appendix 2 Homer gene sequence amplified from U87 mRNA and its
corresponding aminoacid sequence

A TGGGGGAGCA ACCTATCTTC AGCACTCGAG CTCATGTCTT CCAAATTGAC
CCAAACACAA AGAAGAACTG GGTACCCACC AGCAAGCATG CAGTTACTGT GTCTTATTTC
TATGACAGCA CAAGAAATGT GTATAGGATA ATCAGTTTAG ATGGCTCAAA GGCAATAATA
AATAGTACCA TCACCCCAAA CATGACATTT ACTAAAACAT CTCAGAAGTT TGGCCAGTGG
GCTGATAGCC GGGCAAACAC CGTTTATGGA TTGGGATTCT CCTCTGAGCA TCATCTTTCG
AAATTTGCAG AAAAGTTTCA GGAATTTAAA GAAGCTGCTC GACTAGCAAA GGAAAAATCA
CAAGAGAAGA TGGAACTTAC CAGTACACCT TCACAGGAAT CCGCAGGCGG GGATCTTCAG
TCTCCTTTAA CACCAGAAAG TA

MGEQPIFSTR AHVFQIDPNT KKNWVPTSKH AVTVSYFYDS TRNVYRIISL DGSKAIINST
ITPNMTFTKT SQKFGQWADS RANTVYGLGF SSEHHLSKFA EKFQEFKEAA RLAKEKSQEK
MELTSTPSQE SAGGDLQSPL TPES

Appendix 3. Homer gene sequence amplified from rat astrocyte mRNA and
its corresponding amino acid sequence

ATGGGGGA ACAACCTATC TTCAGCACTC GAGCTCATGT CTTCCAGATC GACCCAAACA
CAAAGAAGAA CTGGGTACCC ACCAGCAAGC ATGCAGTTAC TGTGTCTTAT TTCTATGACA
GCACAAGGAA TGTGTATAGG ATAATCAGTC TAGACGGCTC AAAGGCAATA ATAAATAGCA
CCATCACTCC AAACATGACA TTTACTAAAA CATCTCAAAA GTTTGGCCAA TGGGCTGATA
GCCGGGCAAA CACTGTTTAT GGACTGGGAT TCTCCTCTGA GCATCATCTC TCAAAATTTG
CAGAAAAGTT TCAGGAATTT AAAGAAGCTG CTCGGCTGGC AAAGGAGAAG TCGCAGGAGA
AGATGGAACT GACCAGTACC CCTTCACAGG AATCAGCAGG AGGAGATCTT CAGTCTCCTT
TAACACCAGA

MGEQPIFSTR AHVFQIDPNT KKNWVPTSKH AVTVSYFYDS TRNVYRIISL DGSKAIINST
ITPNMTFTKT SQKFGQWADS RANTVYGLGF SSEHHLSKFA EKFQEFKEAA RLAKEKSQEK
MELTSTPSQE SAGGDLQSPL TP
 
Appendix 4 Homer gene sequence amplified from CHO cells mRNA and its
corresponding amino acid sequence

TTCAGCACTC GAGCTCATGT CTTCCAGATT GACCCAAACA CAAAGAAGAA CTGGGTACCC
ACCAGCAAGC ATGCAGTTAC TGTATCTTAT TTTTATGACA GCACAAGAAA TGTATATAGG
ATAATCAGTT TAGATGGCTC AAAGGCAATA ATAAATAGCA CCATCACTCC AAACATGACA
TTTACTAAAA CATCTCAAAA GTTTGGCCAG TGGGCTGATA GCCGGGCAAA TACTGTTTAT
GGATTGGGAT TCTCCTCTGA GCATCATCTT TCCAAATTTG CAGAAAAGTT TCAGGAATTT
AAAGAAGCTG CTCGTCTTGC AAAGGAGAAG TCACAGGAGA AGATGGAACT GACCAGTACA
CCTTCACAGG AATCAGCAGG TGGAGATCTT CAGTCTCCTT TAACACCGAA AGGT

FSTRAHVFQI DPNTKKNWVP TSKHAVTVSY FYDSTRNVYR IISLDGSKAI INSTITPNMT
FTKTSQKFGQ WADSRANTVY GLGFSSEHHL SKFAEKFQEF KEAARLAKEK SQEKMELTST
PSQESAGGDL QSPLTPKG
 Ebadi M. And Srinivasan S.K. 1995 Pharmacological Reviews 47: 575-604
Brakeman P.R., Lanahan A.A., O'Brien R., Roche K., Bames C.A., Huganir R.L., and Worley P.F. 1997 Nature 386: 284-288
Ozawa K.A., Saitoh F., Hirai K., Inokuchi K. 1997 FEBS Letters 412: 183-189
Wright I. And Woodruff P. 1995 CNS Drugs 3: 126-144
Köpfel T. And Gasparinin F. 1996 Drug Development Today 1: 103-108
Ponting C.P. And Phillips C. 1995 Trends in Biochemistry 20: 102-103
Reynolds G.P. And Czudek C. 461-503
Gratz S.S. And Simpson G.M. 1994 CNS Drugs 2: 429-439
Seeman P. 1987 Synapse 1: 133-152
Fatemi H., Meltzer H.Y. And Roth B.L. 1996 In Anti-psychotics Ed. Csernansky J.G. 77-115
Deutch A.Y. 1996 In Anti-psychotics Ed. Csernansky J.G. 117-161
Garcia-Ladona, F.J., Palacios, J.M., and Mengod G.. 1994 Molecular Brain Research 21: 75-84
Garcia-Ladona F.J., Huss Y., Frey P., and Ghandour M.S. 1997 J. Neuroscience Research 50: 50-61
Garcia-Ladona F.J., Claro, E., Garcia, A., and Picatoste, F. 1993 Neuroreport 4: 691-694
Garcia-Ladona F.J., De Barry, J., Girard, C., and Gombos G. (1991) Ectopic granule cell 1991 layer in Exp. Brain Research 86: 90-96 .
Lipska B.K., Jaskiw G.E., and Weinberger D.R. 1993 Neuropsychopharmacology 9: 67-75
Swerdlow N.R., Braff D.L., Bakshi V.P., and Geyer M.A. 1996 In Anti-psychotics Ed. Csernansky J.G. 289-307
J.T August 1997 Gene Therapy in Advances in Pharmacology 40 Academic Press
H.G. Vogel and W.H. Vogel Eds. 1997 Drug Discovery and Evaluation

## Claims

1. A method for treatment of neuroleptic induced disorders or psychosis in a human being comprising administering to said human being a composition comprising an effective amount of a compound which interacts with metabotropic receptors.

2. A method according to claim 1, whereby the interaction effects an inhibition of the metabotropic receptor.

3. A method according to claim 1, whereby the compound is 2-methyl-6- (2-phenylethenyl) pyridine or 2-methyl-6- (phenylethynyl) pyridine hydrochloride.

4. A method for treatment of neuroleptic syndrome or psychosis in a human being comprising administering to said human being a composition comprising an effective amount of a homer expression modifying compound.
